# EUROPEAN PATENT SPECIFICATION

(11) **EP 2 695 641 B1**
(45) Date of publication and mention of the grant of the patent: **03.05.2023**
(21) Application number: 12191534.2
(22) Date of filing: 07.11.2012
(51) Int. Cl.: A62B 23/02, A61M 16/06

(54) **Mask**
Maske
Masque

(30) Priority: 06.08.2012 JP 2012173948
(43) Date of publication of application: 12.02.2014
(73) Proprietor: SAN-M Package Co., Ltd., Shimada-City, Shizuoka 428-0009 (JP)
(72) Inventor: Yagi, Takahiro, Shimada-City, Shizuoka 428-0009 (JP)
(74) Representative: DREISS Patentanwälte PartG mbB

(56) References cited:
- WO-A1-2012/097762
- JP-A- 2011 194 067
- US-A1- 2004 237 964
- US-A1- 2010 051 032

## Description

### BACKGROUND

### Technical Field

The present invention relates to a mask according to the preamble of claim 1.

### Related Art

A mask is used for the purpose of suppressing the inhalation of viruses, bacteria, and pollen, and suppressing the spread of viruses and bacteria along with saliva by a bacterial carrier.

As such a mask, there is a non-woven mask in which two or more non-woven fabrics, including thermally fusible synthetic fibers or natural fibers, that are substantially superimposed by making the horizontal directions thereof the front-back direction, and which has an annular shape through joining the non-woven fabrics at the front side edge portions and the back side edge portions. The front side edge portions and the vicinities of the mask are formed so as to cover the required parts of the face of a wearer, and the back side edge portions and vicinities thereof are formed so as to be stretched around the head of the wearer. The required ranges in the vertical direction at the joining part in the back side edge portions extend further backward than the remaining parts of the back side edge portions positioned above and below the ranges. The synthetic fiber fabrics included in the two or more non-woven fabrics, the inner surfaces of which face each other at the extending parts, are melted and then solidified (Japanese Patent No. 3664543).

However, if the mask deviates from the original position while wearing due to the motions of the mouth and face of a wearer when he or she coughs or talks and a gap is generated between the mask and the face of the wearer, air leaks from the gap. For this reason, the effect of suppressing the inhalation of viruses, bacteria, and pollen and spread of the viruses and bacteria cannot be sufficiently achieved.

In order to suppress the deviation of the mask due to the motions of the mouth and the face of the wearer and suppress the generation of a gap between the mask and the face of the wearer, the following method has been examined.

For example, a surgical mask with cords is provided with a nose grip portion, in which an aluminum or iron core is embedded, at a part of the upper edge of the mask main body with which the nose of the wearer is in contact. The nose grip portion is bent along the nasal bridge of the wearer so as to be in close contact with the nasal bridge of the wearer as well as the mask cord is tied tightly when the mask is worn in order to suppress deviation of the mask and suppress the occurrence of a gap between the mask and the face of the wearer.

However, by only tightly tying the cords along with providing the nose grip portion at an upper edge portion of the mask main body, the mask deviates in some cases during conversation while worn, therefore, it is desired to improve this point. In addition, there is also a problem in that the motions of the neck and head are restricted and it becomes difficult to perform surgical operations if the mask main body is fixed to the face of the wearer by tightly tying the cords.

In addition, a method for preventing the occurrence of deviation between the face and the mask by applying an adhesive or arranging an adhesive tape at the nose grip portion has also been examined.

However, there is a problem in that skin disorders may occur or a strong irritation is felt on the skin when the wearer takes the mask off if the mask is fixed directly to the skin of the wearer's face with adhesive.

Furthermore, a mask in which a leakage preventing member is arranged in the periphery of the nose grip portion in order to suppress leakage of breath and fogging of glasses has also been developed. However, since even a mask of this type cannot sufficiently follow the motions of the mouth and the face during conversation, sneezing, or the like, there is a problem in that the mask deviates from a predetermined position, and as a result, the leakage of breath cannot be sufficiently suppressed.

Although a mask with leakage preventing members provided at both side edge portions of the mask main body has also been developed, the mask also has an insufficient property of following the motions of the mouth and the face, and there is a disadvantage in that the mask deviates from a predetermined position when worn for a long time. JP 2011 194067 A discloses a mask which comprises a mask main body, a leakage prevention part attached to this mask main body and a wearing belt used for mask wearing. US 2004/237964 A1 discloses fold-flat personal respiratory protection devices that have a flat substantially rectangular central portion having first and second edges. US 2010/051032 A1 relates to a four-sided dustproof mask including an upper filter adapted to surround a wearer's nose area. WO 2012/097762 A1 relates to a face mask for removal of biological and mechanical impurities from breathed and/or exhaled air.

The present invention has been made in order to solve the above problems, and an object thereof is to provide a mask capable of suppressing deviation from the original position while worn due to the motions of the mouth and face of a wearer.

### SUMMARY

A first embodiment of the present invention for achieving the above object relates to a mask including: a mask main body; cords that are stretched around both ears or a head of a wearer to fix the mask main body at a predetermined position on a face of the wearer; and a nose grip portion that stands out toward the face of the wearer from a surface of the mask main body at a side in contact with the face of the wearer and covers a nasal bridge of the wearer from above when the mask is worn, wherein leakage preventing members that are capable of standing out from the surface at the side in contact with the face of the wearer are formed at both lateral edge portions of the mask main body, wherein the leakage preventing members are superimposed with the nose grip portion and are formed at both lateral edge portions of the mask main body so as to extend in the direction toward the back surface of the mask main body and stand out from and recline against the back surface. The nose grip portion is formed by folding an upper edge of the mask main body toward the back surface of the mask main body, and by forming a weld line along the upper edge of the mask main body.

In the mask of the above embodiment, the nose grip portion is configured to stand out from the surface of the mask main body at the side in contact with the face of the wearer. In addition, the nose grip portion covers the nasal bridge of the wearer from above when the wearer at the sides of the ears and the mask main body is blocked by the leakage preventing member, and accordingly, it is possible to suppress air leakage from the face of the wearer at the sides of the ears.

A second embodiment of the present invention for achieving the above object relates to the mask of the first embodiment wherein an upper portion of the nose grip portion is attached to an upper edge portion of the mask so that the nose grip portion is capable of standing out from and reclining against the mask main body.

In the mask of the second embodiment, since the nose grip portion can be brought into a state in which the nose grip portion reclines against the mask main body when the mask is not worn, it is possible to suppress deformation and damage of the nose grip portion by being pressed toward the mask main body when the mask is not worn.

A third embodiment of the present invention for achieving the above object relates to the mask of the first or the second embodiment wherein the nose grip portion is formed in a bag shape, and a flexible core material is disposed inside.

In the mask of the third embodiment, since the nose grip portion is formed in a bag shape, and the flexible core material is disposed inside, it is possible to bring the nose grip portion into tight contact with the nasal bridge by bending the nose grip portion along the nasal bridge when the mask is worn.

A fourth embodiment of the present invention for achieving the above object relates to the mask of any of the first to fourth embodiments wherein a nose contact portion that is formed from any one of urethane foam, a non-woven fabric sheet, a laminated non-woven fabric, or a film is provided at a surface of the nose grip portion at a side in contact with a nasal bridge of the wearer.

In the mask of the fourth embodiment, since the nose contact portion is provided on the surface of the nose grip portion in contact with the nasal bridge of the wearer, adhesion between the nose grip portion and the nasal bridge of the wearer is further improved.

A fifth embodiment of the present invention for achieving the above object relates to the mask of any of the second to fifth embodiments wherein: the mask main body is capable of being folded when the mask is not worn and being unfolded to a predetermined shape when the mask is worn; and the mask main body is provided with a shape holding member that maintains an unfolded shape of the mask main body when the mask main body is unfolded.

In the mask of the fifth embodiment, the unfolded shape of the mask main body is held by the shape holding member provided in the mask main body when the mask main body is unfolded. Accordingly, the mask main body is not crushed due to ambient air pressure even when the inside of the mask is decompressed as in a case in which the wearer breathes in while wearing the mask, and a space is secured between the mouth and the face of the wearer and the mask main body. Therefore, it is possible to provide a mask with which the wearer can easily talk and breathe as compared with a case in which the shape holding member is not provided in the mask main body.
Further embodiments of the invention are defined in the dependent claims.

### BRIEF DESCRIPTION OF THE DRAWINGS

Exemplary embodiments of the present invention will be described in detail based on the following figures, wherein:
Fig. 1 is a plan view viewed from a back side and showing a mask according to a first exemplary embodiment that is in a folded state;
Fig. 2 is a cross-sectional view along a plane X-X in Fig. 1 showing the mask according to the first exemplary embodiment in the folded state;
Fig. 3 is a plan view seen from the rear of the mask according to the first exemplary embodiment in a state in which a mask main body is extended and a nose grip portion is made to stand;
Fig. 4 is a perspective view seen obliquely from the left anterior and showing a state in which a wearer wears the mask according to the first embodiment;
Fig. 5 is a perspective view seen obliquely from upper anterior and showing a wearer wearing the mask according to the first embodiment;
Fig. 6 is a plan view seen from the rear of the mask of the first exemplary embodiment wherein a nose contact portion is provided at the nose grip portion showing a state in which the mask main body is extended and the nose grip portion is stood out;
Fig. 7 is a plan view seen from the rear of the mask of a second exemplary embodiment in a folded state;
Fig. 8 is a plan view seen from the front of the mask of the second exemplary embodiment in the folded state;
Fig. 9 is a cross-sectional view along a plane X-X in Fig. 6 showing the mask according to the second exemplary embodiment in the folded state; and
Fig. 10 is a perspective view seen obliquely from the left anterior showing a wearer wearing the mask according to the second exemplary embodiment.

### DETAILED DESCRIPTION

The invention will be now described herein with reference to illustrative embodiments. Those skilled in the art will recognize that many alternative embodiments can be accomplished using the teachings of the present invention and that the invention is not limited to the embodiments illustrated for explanatory purposes.

### 1. First Exemplary Embodiment

Hereinafter, an example of a mask according to the present invention will be described with reference to the drawings.

### <Constitution>

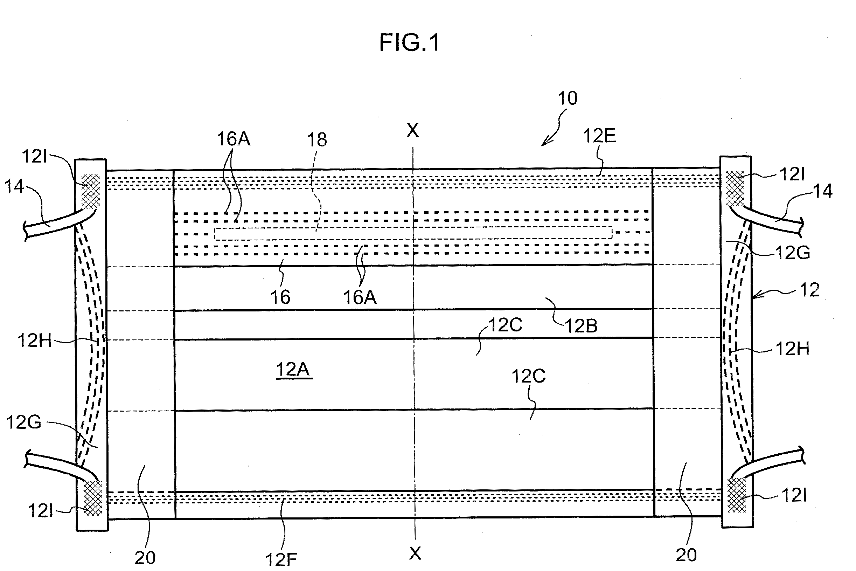

As shown in Figs. 1 to 5, a mask 10 according to a first exemplary embodiment comprises a mask main body 12 that covers the nose and mouth of a wearer 100 when the mask 10 is worn, a pair of elastic cords 14 that are provided at the upper and lower ends of both lateral edges of the mask main body 12 to maintain the mask main body 12 at a predetermined position on the face of the wearer 100 when the mask 10 is worn, a nose grip portion 16 that stands out from a back surface 12A of the mask main body 12, namely a surface on a side that is brought into contact with the face of the wearer 100 toward the face (nasal bridge) of the wearer 100 when the mask 10 is worn and can be bent along the nasal bridge of the wearer, and a leakage preventing member 20 that is superimposed with the nose grip portion 16 from both side edge portions of the mask main body 12, in other words, from a pair of vertical lateral edge portions in the direction toward the back surface 12A of the mask main body 12 and stands out from and recline against the back surface 12A. In addition, the leakage preventing member 20 is not necessarily required in the mask 10 and can be omitted.

As shown in Fig. 1, the mask main body 12 has as a whole a rectangular shape that is long from side to side and is configured by non-woven fabric sheets superimposed with each other in a predetermined number, for example, in three to seven, particularly in five to seven. In addition, the number and the type of the non-woven fabric sheets configuring the mask main body 12 can be determined differently depending on a performance required for the mask 10.

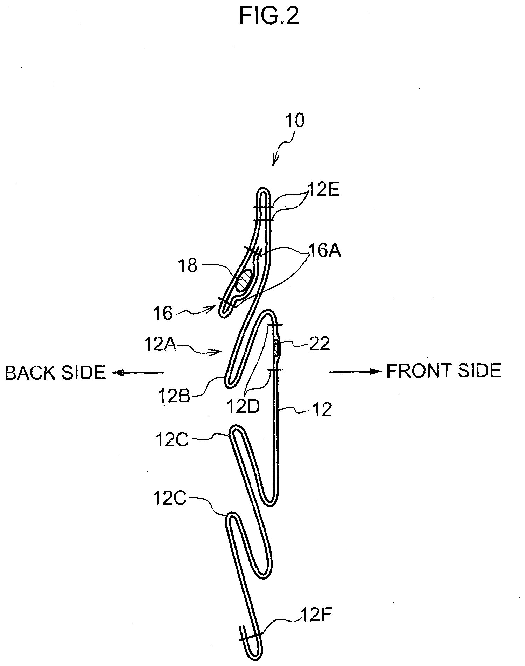

As shown in Figs. 2 to 5, an elastic plate 22 that is a plate formed from an elastic material such as metal or synthetic resin is embedded at the center of the mask main body 12 in the horizontal direction, namely the longitudinal direction of the mask main body 12. In addition, an elastic bar that is a bar formed from the elastic material may be used instead of the elastic plate 22. Both the elastic plate 22 and the elastic bar are examples of a shape holding member in the present invention. Weld lines 12D are formed on both sides of the elastic plate 22 in the mask main body 12, and the elastic plate 22 is thereby held at a predetermined position in the mask main body 12.

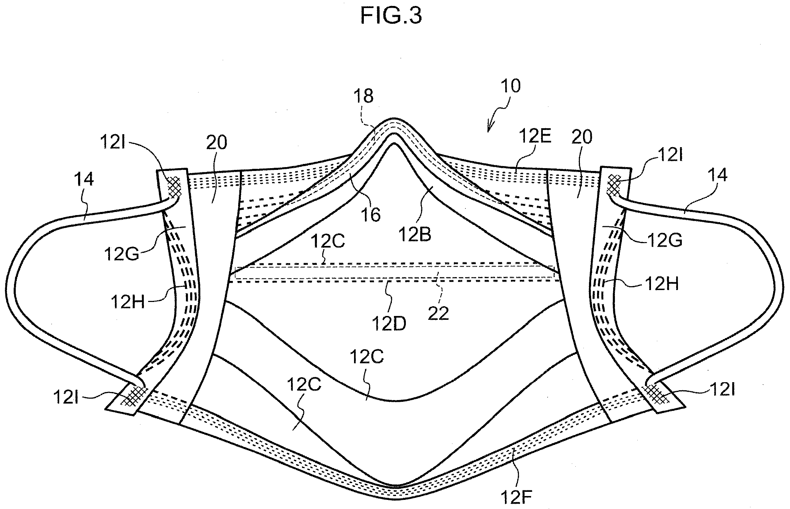

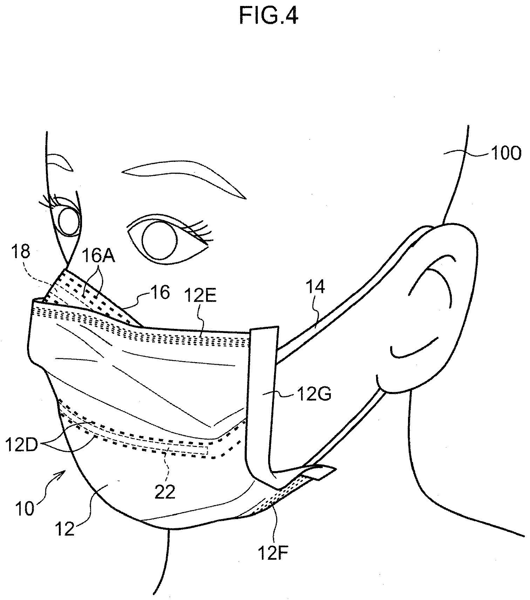

As shown in Fig. 2, a total of three pleated portions that are folded in the longitudinal direction such that convexities are formed on the back side, namely the side facing the face of the wearer 100 when the mask 10 is worn and concavities are formed on the front side are formed in the mask main body 12 such that one of them is formed above the center of the mask main body 12, namely the position at which the elastic plate 22 is embedded and two of them are formed below the center. Among the three pleated portions, a pleated portion 12B above the center of the mask main body 12 is folded downward in Fig. 2, and pleated portions 12C below the center of the mask main body 12 are folded upward in Fig. 2. When the mask 10 is worn, the mask main body 12 is unfolded to a cup shape as shown in Fig. 4 by unfolding forward the pleated portion 12B and the pleated portions 12C as shown in Fig. 3.

As shown in Fig. 2, the mask main body 12 is folded toward the back surface 12A at the upper edge, and a weld line 12E is formed along the upper edge portion. Thus, the nose grip portion 16 is formed. As shown in Figs. 1 and 2, the nose grip portion 16 in a state in which the mask main body 12 is folded is formed so as to lie downward in Figs. 1 and 2 against the back surface 12A such that a part thereof is superimposed with the pleated portion 12B. In addition, the weld line 12E is preferably formed into a reverse V shape in which the center thereof is located at the highest position since the nose grip portion 16 is made to easily stand out from the mask main body 12A when the mask main body 12A is unfolded.

As shown in Figs. 1 to 5, a nose fitting plate 18 that is a plate-shaped member with a width from about 3 to 5 mm formed of a bendable material such as soft steel or aluminum is embedded in the nose grip portion 16. In addition, in place of the nose fitting plate 18, a nose fitting wire that is a line member formed of the bendable material may be embedded. Moreover, the nose fitting plate 18 and the nose fitting wire are examples of a flexible core member in the present invention.

In the nose grip portion 16, two weld lines 16A are formed along both sides of the nose fitting plate 18. Thus, the nose grip portion 16 is formed into a bag shape that contains the nose fitting plate 18 therein and extends in the horizontal direction. In addition, the nose fitting plate 18 is held at a predetermined position inside the nose grip portion 16.

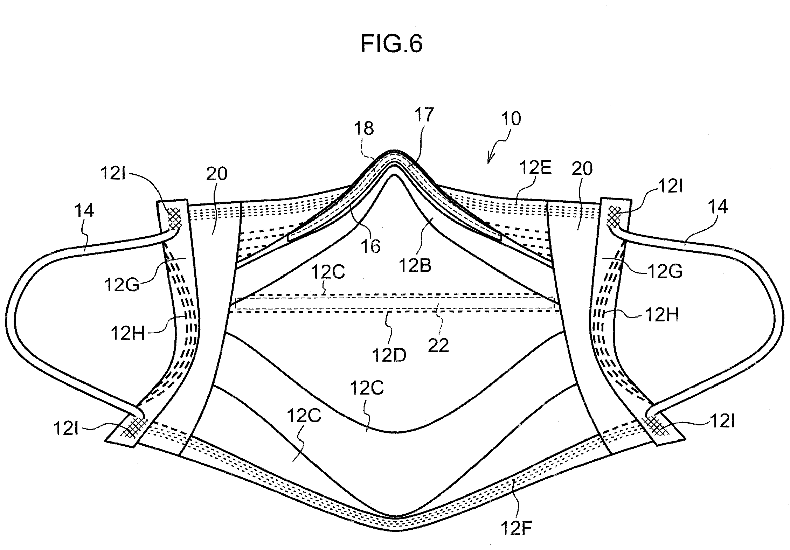

As shown in Fig. 6, a nose contact portion 17 may be provided on the face of the nose grip portion 16 that is in contact with the nasal bridge of the wearer 100 when the mask 10 is worn. The nose contact portion 17 is provided for improving adhesion between the nose grip portion 16 and the nasal bridge of the wearer 100 and can be formed from urethane foam, a non-woven fabric sheet, a laminated non-woven fabric, or the like. In addition, it is possible to further effectively suppress air leakage from the nose grip portion 16 by disposing at the nose contact portion 17 a material such as a film that substantially does not transmit air therethrough.

The lower edge of the mask main body 12 is also folded toward the back surface 12A and welded along a weld line 12F.

As shown in Figs. 1, 3, and 4, reinforced bands 12G are provided at both lateral edges of the mask main body 12 from the back surface 12A to the surface on the front side. One of the edge portions of the leakage preventing member 20 is wrapped with the mask main body 12 in the reinforced bands 12G. An arc-shaped weld line 12H that is curved toward the center of the mask main body 12 is formed at the reinforced bands 12G, and thus, the leakage preventing member 20 is integrally fixed to the mask main body 12 with the weld line 12H. In addition, one end of each rubber cord 14 is welded to the upper end portion of the reinforced band 12G, and the other end thereof is welded to the lower end portion of the reinforced band 12G, with weld portions 121. In addition, the upper edge and the lower edge of the leakage preventing member 20 are fixed to the mask main body 12 with the weld line 12E and the weld line 12F, respectively. In so doing, the leakage preventing member 20 is formed into a bag shape that opens toward the center of the mask main body 12 at the inner side edges. In addition, tie loops may be used instead of the elastic cords 14 in the mask according to the first exemplary embodiment. When the tie loops are used instead of the elastic cords 14, the tie loops may be fixed one by one to each of the lateral edges of the mask main body 12 so as to extend in the vertical direction of the mask. The tie loops are fixed to the side edge portions of the mask main body 12 at the center thereof.

In addition, it is possible to substantially prevent air leakage from the leakage preventing member 20 and improve the adhesion between the leakage preventing member 20 and the face of the wearer 100 by disposing a material such as a film that substantially does not transmit air therethrough, urethane foam, a non-woven fabric sheet, a laminated non-woven fabric, or the like on the leakage preventing member 20.

### <Functions>

As shown in Fig. 2, in a state in which the mask main body 12 is folded, the nose grip portion 16 is positioned below the upper edge of the mask main body 12 and above the pleated portion 12B on the back surface 12A of the mask main body 12 and is formed into a bag shape that extends in the horizontal direction. Accordingly, when the pleated portion 12B and the pleated portions 12C are unfolded forward as shown in Fig. 3, the nose grip portion 16 is brought into a state in which the nose grip portion 16 stands backward from the mask main body 12. In addition, since the leakage preventing member 20 is formed so as to overlap with the nose grip portion 16 as described above, the leakage preventing member 20 also stands backward from the mask main body 12 when the nose grip portion 16 stands.

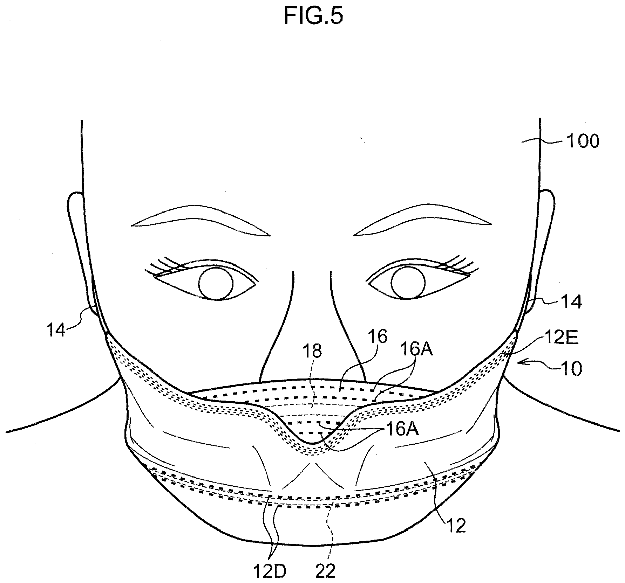

When the wearer 100 wears the mask 10, the mask main body 12 is unfolded as shown in Fig. 3, and the two elastic cords 14 are then respectively stretched around the back sides of ears or the back of the head as shown in Figs. 4 and 5, and the position of the nose grip portion 16 is matched with the nasal bridge. If the position of the nose grip portion 16 is matched with the nasal bridge, the nose grip portion 16 is made to bend along the nasal bridge such that the nose grip portion 16 is brought into tight contact with the nasal bridge. In addition, if the tie loops are used instead of the elastic cords 14 in the mask 10, the upper end portion of one flat cord may be tied with the upper end portion of the other flat cord at the back of the head, and the lower end portion of one flat cord may be tied with the lower end portion of the other flat cord at the back of the neck.

As shown in Figs. 4 and 5, the nose grip portion 16 is at a position on the back side of the mask main body 12, in other words, at a position at which the nose grip portion 16 stands toward the face side of the wearer 100 in a state in which the mask 10 is worn. Accordingly, it is possible to suppress directly transferring the motions of the mouth and the face of the wearer 100 to the nose grip portion 16 through the mask main body 12 and to thereby suppress the displacement of the nose grip portion 16 from the original position when the wearer 100 talks, sneezes, or coughs while wearing the mask 10. In so doing, positional deviation of the mask 10 due to conversation, coughing, or sneezing, leakage of breath due to the positional deviation of the mask 10, and inhalation of air containing viruses, bacteria, or the like are suppressed.

In addition, since the elastic plate 22 extending in the horizontal direction is embedded at the center of the mask main body 12, a football-like unfolded shape of the mask main body 12 is held by the elastic plate 22 as shown in Figs. 4 and 5 when the mask 10 is worn, and decompression and sinking of the mask main body 12 can be suppressed when the wearer 100 breathes in. Accordingly, a state in which a space is formed between the mask main body 12 and the face of the wearer 100 is maintained when the wearer 100 breathes in or out, and therefore, the wearer 100 can more easily talk or breathe compared with a case when wearing a mask that does not have the elastic plate 22.

Furthermore, by providing the nose contact portion 17 at the nose grip portion 16, adhesion between the nose grip portion 16 and the nasal bridge of the wearer 100 can be improved.

In addition, since the leakage preventing member 20 is provided from both side edge portions of the mask main body 12 toward the back surface 12A, leakage of breath and inhalation of air or the like through a gap between the side edge portions of the mask main body 12 and the ear sides of the face of the wearer 100 can be suppressed.

Moreover, since the nose grip portion 16 is configured so as to be folded on the back surface 12A of the mask main body 12 when the mask 10 is not worn, when the mask 10 is not worn, deformation or damage of the nose grip portion 16 can be suppressed when the nose grip portion 16 is pressed.

### 2. Second Exemplary Embodiment

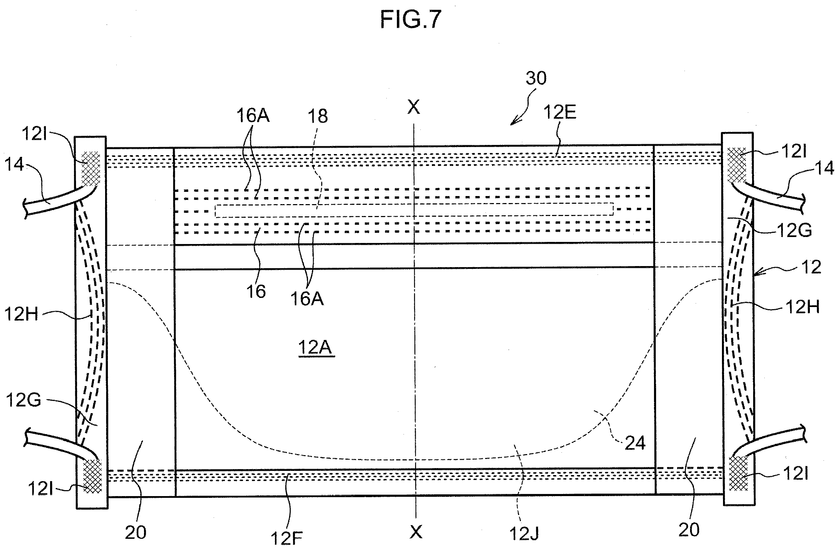

Hereinafter, another example of a mask according to the present invention will be described. In Fig. 7 and subsequent drawings, same reference numerals as those in Figs. 1 to 6 represent the same configuration elements as those represented by the reference numerals in Figs. 1 to 6 unless explicitly stated otherwise.

### <Configuration>

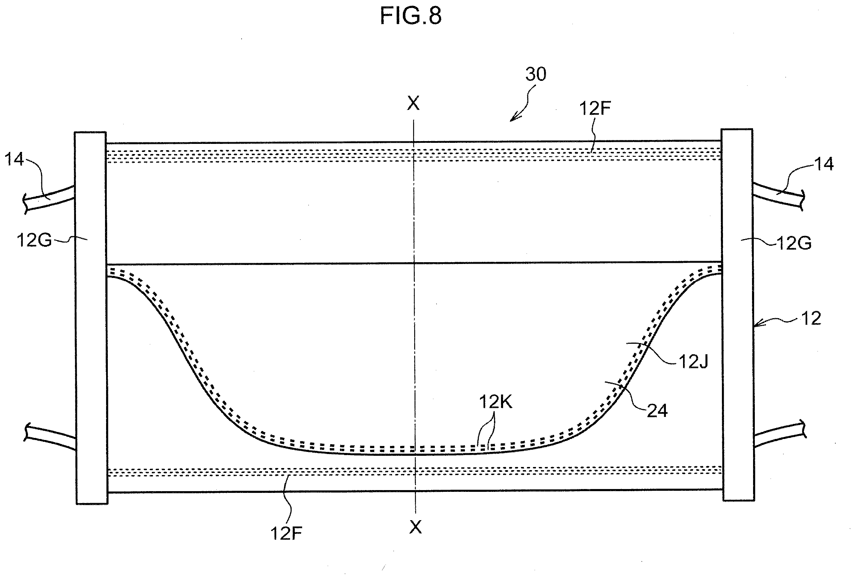

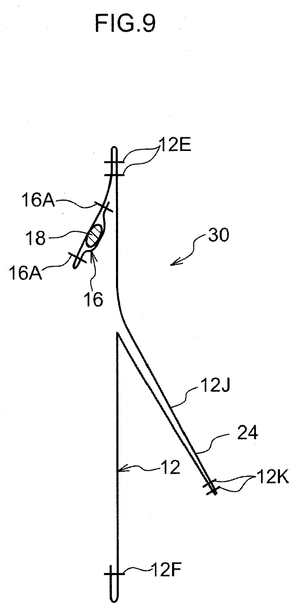

As shown in Figs. 7 to 9, the mask main body 12 in a mask 30 according to a second exemplary embodiment has a horizontally long rectangular shape as a whole and is configured by non-woven fabric sheets superimposed with each other in a predetermined number, for example, three to seven, particularly five to seven, and a pleated portion 12J is formed so as to be folded back such that convexities are formed on the front side as shown in Figs. 8 and 9. The pleated portion 12J is welded in a substantially U shape along a weld line 12K from both end portions toward the center, and a beak-shaped portion 24 with a duck-billed shape is formed by cutting the outside of the pleated portion 12J along the weld line 12K. Accordingly, the mask 30 is also referred to as a so-called bird type mask.

In the mask 30, the mask main body 12 has the same configuration as that of the mask 10 according to the first exemplary embodiment except for the aforementioned points. In addition, the configuration of the nose grip portion 16 is as described above in the first embodiment. Furthermore, tie loops can be used instead of the elastic cords 14 in the same manner as in the mask 10 according to the first exemplary embodiment.

### <Functions>

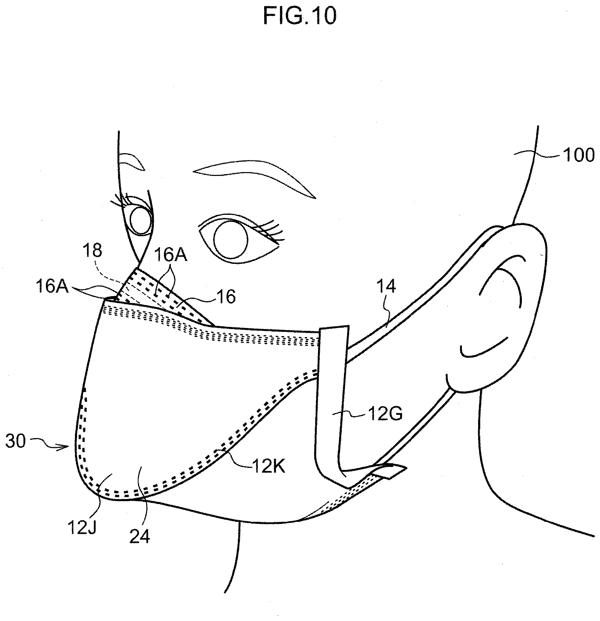

As shown in Fig. 10, when the wearer 100 wears the mask 30, the beak-shaped portion 24 is vertically expanded from the back surface 14A. Thus, the beak-shaped portion 24 protrudes forward in a duck-billed shape, and the nose grip portion 16 is brought into a state in which the nose grip portion 16 stands backward from the mask main body 12. At the same time, the leakage preventing member 20 also stands backward from the mask main body 12.

Next, the two elastic cords 14 are respectively stretched around the back portions of the ears or the back of the head to wear the mask 30, and the position of the nose grip portion 16 is matched with the nasal bridge. If the nose grip portion 16 is matched with the nasal bridge, the nose grip portion 16 is made to bend along the nasal bridge such that the nose grip portion 16 is brought into tight contact with the nasal bridge. In addition, the nose and mouth of the wearer are substantially completely covered with the beak-shaped portion 24 as shown in Fig. 10.

The mask 30 has the following advantages in addition to the advantages of the mask 10 according to the first embodiment. That is, since the shape that the beak-shaped portion 24 protrudes in a duck-billed shape is held by the weld line 12K in a state in which the beak-shaped portion 24 is unfolded in the vertical direction, a space is formed between the beak-shaped portion 24 and the face of the wearer when the mask 30 is worn. Accordingly, the wearer can easily talk and breathe in the same manner as in the mask 10 according to the first embodiment. In addition, it is not necessary to provide a shape holding member such as the elastic plate 22 for maintaining the unfolded shape, unlike the mask 10 according to the first exemplary embodiment.

### EXAMPLES

### 1. Examples 1 and 2 and Comparative Examples 1 and 2

Deviation of a mask of the first exemplary embodiment in which the elastic cords are replaced with tie loops (Example 1), a mask of the first exemplary embodiment (Example 2), a conventional surgical mask with tie loops (Comparative Example 1) and a conventional mask with elastic cords (Comparative Example 2) were evaluated during quiet conversation, conversation with frequent mouth motions, and sneezing (coughing). Here, "quiet conversation" means face-to-face conversation in which the mouth is moved at a level of everyday conversation, and the "conversation with frequent mouth motions" means loud conversation in which the mouth is intentionally moved in a large motion.

The surgical mask with the cords in Comparative Example 1 is a mask in which the nose grip portion is provided at the upper edge portion of the mask main body, and the mask with the rubber in Comparative Example 2 is a mask in which the tie loops in the surgical mask in Comparative Example 1 are replaced with elastic cords. The results are shown in the following Table 1.

**[Table 1]**

| Motions of mouth and face | Example 1 | Example 2 | Comparative Example 1 | Comparative Example 2 |
|---|---|---|---|---|
| Silent conversation | Not deviated | Not deviated | Hardly deviated | Deviated |
| Conversation with frequent mouth motions | Not deviated | Not deviated | Deviated | Deviated |
| Sneezing (coughing) | Hardly deviated | Hardly deviated | Deviated | Deviated |

In Table 1, the expression that the mask was "not deviated" means that there was no gap formed between the nasal bridge of the wearer and the nose grip portion, the expression that the mask was "Hardly deviated" means that a gap was generated between the nasal bridge of the wearer and the nose grip portion while the mask returned to the original position when the wearer stopped talking or sneezing, and the expression that the mask was "deviated" mean that a gap was generated between the nasal bridge of the wearer and the nose grip portion and the mask did not return to the original position. As shown in Table 1, deviation of the mask was not observed in both the case of the quiet conversation and the case of the conversation with frequent mouth motions in Examples 1 and 2 in which the mask of the first exemplary embodiment or the mask wherein in the mask of the first embodiment the elastic cords are replaced with tie loops were used. In addition, even when the wearer sneezed (coughed), significant deviation of the mask was not observed.

On the other hand, in Comparative Example 1 in which the surgical mask with tie loops provided with the nose grip portion at the upper edge portion of the mask main body was used, even though significant deviation of the mask was not observed in the quiet conversation, deviation of the mask was obviously observed in both the case of the conversation with frequent mouth motions and the case of sneezing (coughing).

In Comparative Example 2, deviation of the mask was obviously observed in all of the cases of the quiet conversation, of conversation with frequent mouth motions, and of sneezing (coughing).

It can be understood from the above results that it is possible to effectively suppress deviation of the mask during conversation, sneezing, and coughing by configuring the mask such that the nose grip portion stands backward from the back surface 12A of the mask main body 12 when the mask is worn.

The foregoing description of the exemplary embodiments of the present invention has been provided for the purpose of illustration and description. It is not intended to be exhaustive or to limit the invention to the precise forms disclosed. Obviously, many modification and variations will be apparent to practitioners skilled in the art. The exemplary embodiments were chosen and described in order to best explain the principles of the invention and its practical applications, thereby enabling others skilled in the art to understand the invention for various embodiments and with the various modifications as are suited to the particular use contemplated. It is intended that the scope of the invention be defined by the following claims.

## Claims

1. A mask (10) comprising:
a mask main body (12);
cords (14) that are stretched around both ears or a head of a wearer to fix the mask main body (12) at a predetermined position on a face of the wearer; and
a nose grip portion (16) that stands out toward the face of the wearer from a surface of the mask main body (12) at a side in contact with the face of the wearer and covers a nasal bridge of the wearer from above when the mask is worn,
wherein leakage preventing members (20) that are capable of standing out from the surface at the side in contact with the face of the wearer are formed at both lateral edge portions of the mask main body,
wherein the leakage preventing members (20) are superimposed with the nose grip portion (16) and are formed at both lateral edge portions of the mask main body (12) so as to extend in the direction toward the back surface (12A) of the mask (10) main body (12) and stand out from and recline against the back surface (12A),
wherein the nose grip portion (16) is formed by folding an upper edge of the mask main body (12) toward the back surface (12A) of the mask main body (12), and by forming a weld line (12E) along the upper edge of the mask main body (12).

2. The mask (10) according to claim 1, wherein an upper portion of the nose grip portion (16) is attached to an upper edge portion of the mask main body (12) so that the nose grip portion 816) is capable of standing out from and reclining against the mask main body (10).

3. The mask (10) according to claim 1 or 2, wherein the nose grip portion (16) is formed in a bag shape, and a flexible core material is disposed inside.

4. The mask (10) according to any one of claims 1 to 3, wherein a nose contact portion (17) that is formed from any one of urethane foam, a non-woven fabric sheet, a laminated non-woven fabric, or a film is provided at a surface of the nose grip portion (16) at a side in contact with a nasal bridge of the wearer.

5. The mask (10) according to any one of claims 2 to 4, wherein:
the mask main body (12) is capable of being folded when the mask is not worn and being unfolded to a predetermined shape when the mask is worn; and
the mask main body (12) is provided with a shape holding member (22) that maintains an unfolded shape of the mask main body (12) when the mask main body is unfolded.

6. The mask (10) according to claim 1, wherein upper and lower ends of the leakage preventing members (20) are fixed to the mask main body (12) by a weld line (12E) along an upper edge of the mask main body (12) and a weld line (12F) along a lower edge of the mask main body (12), respectively, wherein the mask main body (12) comprises a shape holding member (22) which is an elastic plate extending at a center of the mask main body (12) in a horizontal direction so as to maintain an unfolded shape of the mask main body (12) when the mask main body (12) is unfolded, wherein the mask main body comprises a plurality of pleated portions (12B, 12C) extending along a longitudinal direction thereof, and upon extending the mask, the pleated portions (12B and 12C) are unfolded forward, and the mask main body (12) is extended into a forward protruding shape, wherein the pleated portions (12B) are located above a center line of the mask main body (12), and the pleated portions (12C) are located below the center line of the mask main body (12), wherein the pleated portions (12B, 12C) are defined by substantially arc-shaped weld lines (12H) extending from both lateral edges of the mask main body (12) toward a center of the mask main body (12).

## Patentansprüche

1. Maske (10), umfassend:
einen Maskenhauptkörper (12);
Bänder (14), die um beide Ohren oder den Kopf eines Trägers gedehnt werden, um den Maskenhauptkörper (12) an einer vorbestimmten Stelle auf dem Gesicht des Trägers zu fixieren; und
einen Nasengriffteil (16), der von einer Oberfläche des Maskenhauptkörpers (12) auf einer mit dem Gesicht des Trägers in Kontakt stehenden Seite zu dem Gesicht des Trägers hin vorsteht und den Nasenrücken des Trägers von oben bedeckt, wenn die Maske getragen wird,
wobei Austrittsschutzelemente (20), die von der Oberfläche auf der mit dem Gesicht des Trägers in Kontakt stehenden Seite vorstehen können, an beiden seitlichen Randteilen des Maskenhauptkörpers gebildet sind,
wobei die Austrittsschutzelemente (20) über den Nasengriffteil (16) gelegt sind und an beiden seitlichen Randteilen des Maskenhauptkörpers (12) so gebildet sind, dass sie sich in Richtung der Rückfläche (12A) des Hauptkörpers (12) der Maske (10) erstrecken und über die Rückfläche (12A) vorstehen und sich gegen diese anlehnen,
wobei der Nasengriffteil (16) gebildet ist, indem ein oberer Rand des Maskenhauptkörpers (12) zu der Rückfläche (12A) des Maskenhauptkörper (12) hin gefaltet wird und indem eine Schweißlinie (12E) entlang dem oberen Rand des Maskenhauptkörpers (12) gebildet wird.

2. Maske (10) nach Anspruch 1, wobei ein oberer Teil des Nasengriffteils (16) an einem oberen Randteil des Maskenhauptkörpers (12) angebracht ist, sodass der Nasengriffteil (16) in der Lage ist, über den Maskenhauptkörper (10) vorzustehen und sich an diesen anzulehnen.

3. Maske (10) nach Anspruch 1 oder 2, wobei der Nasengriffteil (16) in einer Beutelform gebildet ist und im Inneren ein flexibles Kernmaterial angeordnet ist.

4. Maske (10) nach einem der Ansprüche 1 bis 3, wobei ein Nasenkontaktteil (17), der aus Urethanschaum, einer Vliesstofflage, einem Schichtvliesstoff oder einer Folie gebildet ist, an einer Oberfläche des Nasengriffteils (16) auf der mit dem Nasenrücken des Trägers in Kontakt stehenden Seite vorgesehen ist.

5. Maske (10) nach einem der Ansprüche 2 bis 4, wobei:
der Maskenhauptkörper (12) gefaltet werden kann, wenn die Maske nicht getragen wird, und in eine vorbestimmte Form entfaltet werden kann, wenn die Maske getragen wird; und
der Maskenhauptkörper (12) mit einem Formhalteelement (22) versehen ist, das eine entfaltete Form des Maskenhauptkörpers (12) aufrechterhält, wenn der Maskenhauptkörper entfaltet ist.

6. Maske (10) nach Anspruch 1, wobei obere und untere Enden der Austrittsschutzelemente (20) an dem Maskenhauptkörper (12) durch eine Schweißlinie (12E) entlang einem oberen Rand des Maskenhauptkörpers (12) und eine Schweißlinie (12F) entlang einem unteren Rand des Maskenhauptkörpers (12) jeweils befestigt sind, wobei der Maskenhauptkörper (12) ein Formhalteelement (22) umfasst, das eine sich in der Mitte des Maskenhauptkörpers (12) in einer horizontalen Richtung erstreckende elastische Platte ist, um so eine entfaltete Form des Maskenhauptkörpers (12) aufrechtzuerhalten, wenn der Maskenhauptkörper (12) entfaltet ist, wobei der Maskenhauptkörper eine Vielzahl von Faltenabschnitten (12B, 12C) umfasst, die sich entlang seiner Längsrichtung erstrecken, und wobei beim Auseinanderziehen der Maske die Faltenabschnitte (12B und 12C) nach vorne entfaltet werden und der Maskenhauptkörper (12) in eine nach vorne vorspringende Form erweitert wird, wobei die Faltenabschnitte (12B) oberhalb einer Mittellinie des Maskenhauptkörper (12) angeordnet sind und die Faltenabschnitte (12C) unterhalb der Mittellinie des Maskenhauptkörpers (12) angeordnet sind, wobei die Faltenabschnitte (12B, 12C) durch im Wesentlichen bogenförmige Schweißlinien (12H) definiert sind, die sich von beiden Seitenrändern des Maskenhauptkörpers (12) zu der Mitte des Maskenhauptkörpers (12) hin erstrecken.

## Revendications

1. Masque (10) comprenant :
un corps principal de masque(12) ;
des cordons (14) qui sont tendus autour des deux oreilles ou de la tête d'un porteur pour fixer le corps principal du masque (12) à une position prédéterminée sur le visage du porteur ; et
une partie de prise nasale (16) qui fait saillie vers le visage du porteur à partir d'une surface du corps principal du masque (12) au niveau d'un côté en contact avec le visage du porteur et couvre une arête nasale du porteur par le dessus lorsque le masque est porté,
dans lequel des éléments anti-fuite (20) qui sont capables de faire saillie de la surface du côté en contact avec le visage du porteur sont formés sur les deux parties de bord latéral du corps principal du masque,
dans lequel les éléments anti-fuites (20) sont superposés à la partie de saisie nasale (16) et sont formés au niveau des deux parties de bord latéral du corps principal du masque (12) de manière à s'étendre dans la direction de la surface arrière (12A) du corps principal (12) du masque (10) et à ressortir de la surface arrière (12A) et à s'incliner contre celle-ci,
dans lequel
la partie de prise nasale (16) est formée en pliant un bord supérieur du corps principal du masque (12) vers la surface arrière (12A) du corps principal du masque (12), et en formant une ligne de soudure (12E) le long du bord supérieur du corps principal du masque (12).

2. Masque (10) selon la revendication 1, dans lequel une partie supérieure de la partie de prise nasale (16) est fixée à une partie de bord supérieur du corps principal du masque (12), de sorte que la partie de prise nasale (16) est capable de se détacher du corps principal du masque (10) et de s'incliner contre celui-ci.

3. Masque (10) selon la revendication 1 ou 2, dans lequel la partie de prise nasale (16) est formée en forme de sac, et un matériau central flexible est disposé à l'intérieur.

4. Masque (10) selon l'une quelconque des revendications 1 à 3, dans lequel une partie de contact avec le nez (17) qui est formée à partir d'une mousse d'uréthane, d'une feuille de tissu non tissé, d'un tissu non tissé stratifié, ou d'un film, est prévue sur une surface de la partie de prise nasale (16) sur un côté en contact avec l'arête nasale du porteur.

5. Masque (10) selon l'une quelconque des revendications 2 à 4, dans lequel :
le corps principal du masque (12) est capable d'être plié lorsque le masque n'est pas porté et d'être déplié à une forme prédéterminée lorsque le masque est porté ; et
le corps principal du masque (12) est pourvu d'un élément de maintien de forme (22) qui maintient une forme dépliée du corps principal du masque (12) lorsque le corps principal du masque est déplié.

6. Masque (10) selon la revendication 1, dans lequel les extrémités supérieure et inférieure des éléments anti-fuite (20) sont fixées au corps principal du masque (12) par une ligne de soudure (12E) le long d'un bord supérieur du corps principal du masque (12) et une ligne de soudure (12F) le long d'un bord inférieur du corps principal du masque (12), respectivement, dans lequel le corps principal du masque (12) comprend un élément de maintien de forme (22) qui est une plaque élastique s'étendant au centre du corps principal du masque (12) dans une direction horizontale de façon à maintenir une forme dépliée du corps principal du masque (12) lorsque le corps principal du masque (12) est déplié, dans lequel le corps principal du masque comprend une pluralité de parties plissées (12B, 12C) s'étendant le long d'une direction longitudinale de celui-ci, et lors de l'extension du masque, les parties plissées (12B et 12C) sont dépliées vers l'avant, et le corps principal du masque (12) est étendu en une forme faisant saillie vers l'avant, dans lequel les parties plissées (12B) sont situées au-dessus d'une ligne centrale du corps principal du masque (12), les parties plissées (12B) sont situées au-dessus d'une ligne centrale du corps principal du masque (12), et les parties plissées (12C) sont situées en dessous de la ligne centrale du corps principal du masque (12), dans lequel les parties plissées (12B, 12C) sont définies par des lignes de soudure sensiblement en forme d'arc (12H) s'étendant des deux bords latéraux du corps principal du masque (12) vers un centre du corps principal du masque (12).
